# EUROPEAN PATENT APPLICATION

(11) **EP 3 309 143 A1**
(43) Date of publication of application: **18.04.2018**
(21) Application number: 16194168.7
(22) Date of filing: 17.10.2016
(51) Int. Cl.: C07C 269/04, C07C 68/02, C07C 51/58, C07C 55/40, C07C 69/96, C07C 271/12, C07C 271/20

(54) **METHOD FOR PRODUCING FLUOROFORMATE COMPOUNDS**

(71) Applicant: Solvay SA, 1120 Bruxelles (BE)
(72) Inventor: BUISINE, Olivier, 69230 Saint Genis-Laval (FR); KASUBKE, Michael, 30173 Hannover (DE); SEFFER, Dirk, 30173 Hannover (DE); BOMKAMP, Martin, 30161 Hannover (DE)
(74) Representative: Menville, Laure

(57) **Abstract**

The invention relates to the preparation of fluorosubstituted fluoroformate from halosubstituted haloformate by reaction with at least one fluorinating agent in the presence of a catalyst. The further preparation of fluorosubstituted (fluoro)alkyl carbonates, fluorosubstituted (fluoro)alkyl polycarbonates, fluorosubstituted (fluoro)alkyl carbamates and fluorosubstituted (fluoro)alkyl polycarbamates is disclosed.

## Description

### TECHNICAL FIELD OF THE INVENTION

The invention relates to the preparation of fluorinated compounds, and more specifically to a new improved method for producing fluoroformate compounds.

### TECHNICAL BACKGROUND

Fluoroformate compounds are highly valuable intermediate chemical compounds, useful for the preparation of fluorinated compounds. For instance, fluoroalkyl fluoroformate compounds may be used for preparing fluoroalkyl (fluoro)alkyl carbonates as disclosed in the International Patent Application WO 2011/006822.

It is known by the person skilled in the art that fluoroalkyl (fluoro)alkyl carbonates can be prepared from carbonyl fluoride and the corresponding aldehyde. For example, 1-fluoroethyl fluoroformate CH₃CHF-O-C(O)F can be obtained by reacting carbonyl fluoride COF₂ with acetaldehyde CH₃C(O)H. One issue of this method is that it cannot easily be used at industrial scale. It requires an industrial quantity of carbonyl fluoride, which is lethal at ppm level. Additionally, acetaldehyde is highly flammable. It would be advantageous to provide alternative routes which could be easier to implement at industrial scale.

It is also known from WO 2011/006822 that fluoroformate compounds, especially fluoroalkyl fluoroformates, can be prepared from the respective chloroalkyl chloroformates in a "Halex" type reaction, i.e. substitution of fluorine atoms for the chlorine atoms by fluorinating agents, using a fluorinating reactant like alkali or alkaline earth metal fluorides, for example LiF, KF, CsF, NaF, NH₄F or amine hydrofluorides, or the respective HF adducts. This process could be improved. In particular, it would be interesting to provide a Halex reaction process having a better yield, typically a yield higher than 20%.

One object of the present invention is to provide a new route to manufacture fluoroformate compounds. Said new route does not have the advantageously the drawbacks of the currently known methods. In particular, the new process according to the invention may provide fluoroformate compounds with a yield compatible with industrial use. Advantageously, the process according to the invention may require easily available raw materials. Additionally, the process according to the invention may be used at industrial scale.

### SUMMARY OF THE INVENTION

One subject-matter of the present invention is a process for preparing fluorosubstituted fluoroformate of the formula (II)

FCHR-O-C(O)F (II)

from halosubstituted haloformate of formula (I)

XCHR-O-C(O)X (I)

wherein R represents a hydrogen atom or a substituted or unsubstituted hydrocarbon group having from 1 to 6 carbon atoms, and each X independently represents a halogen atom, with the proviso that at least one X is different from fluorine,
by reacting said halosubstituted haloformate of formula (I) with at least one fluorinating agent, said fluorinating agent being selected from the group consisting of metal fluorides or organic fluorides, in the presence of a catalyst, said catalyst being selected from the group consisting of metal bifluorides or organic bifluorides.

Another subject-matter of the present invention is a process for preparing fluorosubstituted (fluoro)alkyl carbonates of the formula (IV)

FCHR-O-C(O)-OR' (IV)

comprising preparing fluorosubstituted fluoroformate of the formula (II) as described above, and reacting said fluorosubstituted fluoroformate of the formula (II) with an alcohol of formula (III)

R'OH (III)

wherein R' represents a substituted or unsubstituted hydrocarbon group having from 1 to 12 carbon atoms.

Another subject-matter of the present invention is a process for preparing fluorosubstituted (fluoro)alkyl polycarbonates of the formula (VI)

[FCHR-O-C(O)-O-]ₙ-R" (VI)

comprising preparing fluorosubstituted fluoroformate of the formula (II) as described above, and reacting said fluorosubstituted fluoroformate of the formula (II) with a polyol of formula (V)

R"-[-OH]ₙ (V)

wherein n is 2, 3 or 4, and R" represents a substituted or unsubstituted hydrocarbon residue having from 1 to 12 carbon atoms.

Another subject-matter of the present invention is a process for preparing fluorosubstituted (fluoro)alkyl carbamates of the formula (VIII)

FCHR-O-C(O)-NR¹R² (VIII)

comprising preparing fluorosubstituted fluoroformate of the formula (II) as described above, and reacting said fluorosubstituted fluoroformate of the formula (II) with an with an amine of formula (VII),

R¹R²NH (VII)

wherein R¹ and R², independently from each other, represent a substituted or unsubstituted hydrocarbon group having from 1 to 12 carbon atoms.

Another subject-matter of the present invention is a process for preparing fluorosubstituted (fluoro)alkyl polycarbamates of the formula (X)

[FCHR-O-C(O)-NR¹-]ₘ-R"' (X)

comprising preparing fluorosubstituted fluoroformate of the formula (II) as described above, and reacting said fluorosubstituted fluoroformate of the formula (II) with an with a polyamine of formula (IX),

R"'-[-NR¹H]ₘ (IX).

wherein n is 2, 3 or 4, each R¹, independently from each other, represent a substituted or unsubstituted hydrocarbon group having from 1 to 12 carbon atoms, and R" represents a substituted or unsubstituted hydrocarbon residue having from 1 to 12 carbon atoms.

### DESCRIPTION OF THE INVENTION

In the present disclosure, the expression « comprised between ... and ... » should be understood has including the limits.

In the present disclosure, "alkyl" refers to a linear or branched saturated hydrocarbon chain. Preferred examples of alkyl groups are methyl, ethyl, propyl, isopropyl, butyl, isobutyl and t-butyl.

"Alkenyl" refers to a linear or branched saturated hydrocarbon chain, having one or more double bonds. Preferred examples of alkenyl groups are ethenyl, propenyl, isopropenyl, butenyl, and isobutenyl.

"Alkynyl" refers to a linear or branched saturated hydrocarbon chain, having one or more triple bonds. Preferred examples of alkenyl groups are ethynyl, propynyl, isopropynyl, butynyl, and isobutynyl.

"Aryl" refers to a monocyclic or polycyclic aromatic hydrocarbon group. Preferred examples of aryl groups are phenyl and naphthyl. When the group is a polycyclic group, the rings may be condensed or attached by σ (sigma) bonds.

"Halogen" refers to any atom selected from the group consisting of F, Cl, Br and I.

One subject-matter of the present invention is a process for preparing fluorosubstituted fluoroformate of the formula (II) from halosubstituted haloformate of formula (I):

XCHR-O-C(O)X → FCHR-O-C(O)F (I) (II)

According to a first embodiment, R may represent a hydrogen atom. Thus, the process according to the invention may consist in preparing fluoromethyl fluoroformate CH₂FC(O)F from a halomethyl haloformate, and preferably from chloromethyl chloroformate CH₂ClC(O)Cl.

According to a second embodiment, R may represent a substituted or unsubstituted hydrocarbon group having from 1 to 6 carbon atoms. R may preferably be selected from the group consisting of a substituted or unsubstituted alkyl group having from 1 to 6 carbon atoms, a substituted or unsubstituted alkenyl group having from 2 to 6 carbon atoms, a substituted or unsubstituted alkynyl group having from 2 to 6 carbon atoms, and a substituted or unsubstituted aryl group. Alkyl groups, alkenyl groups, alkynyl groups and aryl groups may optionally be substituted one or several times by a halogen atom. More preferably, R may be selected from the group consisting of a linear or branched unsubstituted alkyl group having from 1 to 6 carbon atoms, a linear or branched unsubstituted alkenyl group having from 2 to 6 carbon atoms, and a phenyl group. Even more preferably, R may be selected from the group consisting of CH₃, CH₃CH₂, (CH₃)CH=CH, C(CH₃)₂=CH, CH₂=CH(CH₂), CH₂=CH(CH₂CH₂), CH₂=CH, and phenyl.

In formula (I), each X independently represents a halogen atom, with the proviso that at least one X is different from fluorine. According to one embodiment, both X are different. Therefore, one X can be F and the other X can be Cl, Br or I. According to another embodiment, both X are identical and are Cl, Br or I. Preferably, both X may represent Cl. The halosubstituted haloformate of formula (I) is preferably a chlorosubstituted chloroformate.

According to a preferred embodiment, the halosubstituted haloformate of formula (I) may be selected from the group consisting of 1-chloroethyl chloroformate (CH₃CHCl-O-C(O)Cl), 1-chloromethyl chloroformate (CH₂Cl-O-C(O)Cl), and 1-chlorophenyl chloroformate (C₆H₅-CHCl-O-C(O)Cl). More preferably, the halosubstituted haloformate of formula (I) is 1-chloroethyl chloroformate.

The chlorosubstituted chloroformates may be prepared by any methods known by the person skilled in the art. For example, such compounds may be prepared by reaction between phosgene and an aldehyde as described in US patent 5,712,407.

The process according to the invention consists in reacting said halosubstituted haloformate of formula (I) with at least one fluorinating agent, said fluorinating agent being selected from the group consisting of metal fluorides or organic fluorides, in the presence of a catalyst, said catalyst being selected from the group consisting of metal bifluorides or organic bifluorides.

The fluorinating agent may preferably be a metal fluoride, more preferably an alkali or alkaline earth metal fluoride. Preferably, the fluorinating agent may be selected from the group consisting of LiF, KF, CsF, NaF, MgF₂ and CaF₂, more preferably from the group consisting of KF and NaF, and even more preferably, the fluorinating agent is KF. The fluorinating agent may be another metal fluoride, like copper fluoride CuF₂. Alternatively, the fluorinating agent may be an organic fluoride, like ammonium fluoride (NH₄F).

The catalyst according to the invention is a bifluoride salt. "Bifluoride salt" refers to any salt comprising the anion [HF₂]⁻. The catalyst may preferably be a metal bifluoride, more preferably an alkali or alkaline earth metal bifluoride. Preferably, the fluorinating agent may be selected from the group consisting of LiHF₂, KHF₂, CsHF₂ and NaHF₂, more preferably from the group consisting of KHF₂ and NaHF₂, and even more preferably, the catalyst is KHF₂. It is not excluded that the catalyst could be another metal bifluoride or an organic bifluoride, like ammonium bifluoride ([NH₄][HF₂]). According to a preferred embodiment, the catalyst may be selected according to the fluorinating agent, and it may be an HF adduct of said fluorinating agent. For example, if the fluorinating agent is KF, then the catalyst may be KHF₂.

The catalyst according to the invention may be added as such to the reaction medium, or it may be prepared in situ. The bifluoride catalyst may be prepared in situ by reaction of the fluorinating agent with a Brönsted acid. Said Brönsted acid may preferably have a pK_{A} in water equal to or less than 5. For example, it may be selected from the group consisting of HCl, HF, H₂SO₄, CF₃CO₂H and CH₃CO₂H.

Unexpectedly, it was discovered by the inventors that the halogen exchange ("Halex") reaction necessitates the combination of a fluorinating agent and a bifluoride salt, which plays the role of catalyst of the halogen exchange reaction.

The reaction may be carried out with from 1 to 10 molar equivalents, preferably with from 1.1 to 5 molar equivalents, of fluorinating agent. The mole equivalents are calculated with reference to the number of halogen atom involved in the halogen atom exchange reaction.

Moreover, the reaction may be carried out with from 0.1 to 1.1 molar equivalent, preferably with from 0.25 to 1 molar equivalent, of catalyst.

The reaction may be carried out in an appropriate solvent, preferably in an aprotic solvent. The solvent may be acetonitrile, adiponitrile, valeronitrile, dichloromethane, dichlorobenzene and alkanes. The reaction may preferably be carried out in acetonitrile.

The reaction may preferably be performed batch wise, for example in an autoclave. Alternatively, it can be performed continuously.

The reaction temperature may vary during the reaction. The temperature may preferably be equal or higher than the ambient temperature, more preferably equal or higher than 50°C, even more preferably equal or higher than 70°C. It is preferably performed at a temperature equal to or lower than 300°C, more preferably equal or lower than 200°C, more preferably equal or lower than 100°C. It is preferred to perform the reaction at such an elevated temperature and/or for a sufficient time until the desired conversion has taken place.

It is preferably performed in the liquid phase. The pressure depends from the reaction temperature; the higher the reaction temperature, the higher is the pressure in the reactor. The reaction pressure may vary during the reaction. The reaction can be performed at ambient pressure (about 1 bar absolute). The pressure may preferably be equal or higher than 1 bar (abs.), more preferably equal or higher than 1.5 bar (abs.). Preferably, the reaction is performed at a pressure equal to or lower than 10 bar (abs.). The reaction vessel can be pressurized, if desired, with an inert gas, especially with nitrogen. Preferably, the reaction is carried out under autogenous pressure.

Advantageously, the combined use of a fluorinating agent and a bifluoride catalyst makes it possible to prepare the desired fluorosubstituted fluoroformate with a good yield, preferably a yield equal to or higher than 20%. The process according to the invention is more compatible with the industrial scale than the previously known processes, since the starting materials are more easily available and show less environmental and security issues.

At the end of the halogen exchange reaction, a fluorosubstituted fluoroformate of the formula (II) FCHR-O-C(O)F was prepared. The group R may be as defined above.

According to a preferred embodiment, the fluorosubstituted fluoroformate of formula (II) may be selected from the group consisting of 1-fluoroethyl fluoroformate (CH₃CHF-O-C(O)F), 1-fluoromethyl fluoroformate (CH₂F-O-C(O)F) and 1-fluoro-1-phenyl-methyl fluoroformate (C₆H₅-CHF-O-C(O)F). More preferably, the fluorosubstituted fluoroformate of formula (I) is 1-fluoroethyl fluoroformate.

If desired, said fluorosubstituted fluoroformates can be isolated from the reaction mixture according to methods known in the art, for example by distillation.

The fluorosubstituted formates obtained by the process according to the invention may be used for any purposes. They may be used as chemical intermediates for the preparation of fluorinated compounds. They can be used as fluorinating agent or to introduce a protecting group in aminoacids or peptides.

According to a preferred embodiment, the fluorosubstituted fluoroformates prepared by the process according to the invention may be used in the preparation of fluorosubstituted (fluoro)alkyl carbonates and fluorosubstituted (fluoro)alkyl carbamates.

According to one object of the present invention, fluorosubstituted (fluoro)alkyl carbonates of the formula (IV)

FCHR-O-C(O)-OR' (IV)

may be prepared by reacting a fluorosubstituted fluoroformate of the formula (II) obtained as disclosed above with an alcohol of formula (III)

R'OH (III)

R' represents a substituted or unsubstituted hydrocarbon group having from 1 to 12 carbon atoms. R' may preferably be selected from the group consisting of a substituted or unsubstituted alkyl group having from 1 to 12 carbon atoms, a substituted or unsubstituted alkenyl group having from 2 to 12 carbon atoms, a substituted or unsubstituted alkynyl group having from 2 to 12 carbon atoms, a substituted or unsubstituted aryl group having from 6 to 12 carbon atoms and a substituted or unsubstituted aryl-alkyl group having from 7 to 12 carbon atoms. Alkyl groups, alkenyl groups, alkynyl groups, aryl groups and aryl-alky groups may optionally be substituted one or several times by a halogen atom, by a nitrile group, or by an alkoxy group. More preferably, R' may be selected from the group consisting of a linear or branched alkyl group having from 1 to 7 carbon atoms, a linear or branched alkenyl group having from 2 to 7 carbon atoms, a linear or branched alkynyl group having from 2 to 7 carbon atoms, phenyl and benzyl, each of the alkyl group, the alkenyl group, the alkynyl group phenyl and benzyl being unsubstituted or substituted one or several times by a halogen atom, preferably a fluorine atom, by a nitrile group, or by a methoxy group. Even more preferably, R' may be selected from the group consisting of CH₃, CH₃CH₂, CH≡C-CH₂CH₂, CH≡C-CH₂, N≡C-CH₂, CH₂=C-CH₂, CH₃-O-CH₂CH₂, C₆H₅, C₆H₅-CH₂., CF₃CH₂ and CH₃CHF.

Preferably, the alcohol of formula (III) can be selected from the group consisting of methanol, ethanol, phenol, allyl alcohol (CH₂=CHCH₂OH) and propargyl alcohol (CH≡CCH₂OH).

Similarly, the alcoholysis reaction can be carried out with polyol instead of alcohol. Thus, according to one object of the present invention, fluorosubstituted (fluoro)alkyl polycarbonates of the formula (VI)

[FCHR-O-C(O)-O-]ₙ-R" (VI)

may be prepared by reacting a fluorosubstituted fluoroformate of the formula (II) obtained as disclosed above with a polyol of formula (V)

R"-[-OH]ₙ (V)

wherein n is 2, 3 or 4, and R" represents a substituted or unsubstituted hydrocarbon residue having from 1 to 12 carbon atoms. The valence of the hydrocarbon residue R" depends from the number n: if n is 2, then R" is a bivalent residue; if n is 3, the R" is a trivalent residue; if n is 4, then R" is a tetravalent residue.

The polyol of formula (V) may preferably be a diol (n=2). Accordingly, R" may preferably be selected from the group consisting of a substituted or unsubstituted alkylene group having from 1 to 12 carbon atoms, a substituted or unsubstituted alkenylene group having from 2 to 12 carbon atoms, a substituted or unsubstituted alkynylene group having from 2 to 12 carbon atoms, a substituted or unsubstituted arylene group having from 6 to 12 carbon atoms and a substituted or unsubstituted alkyl-aryl-alkyl group having from 7 to 12 carbon atoms. Akylene groups, alkenylene groups, alkynylene groups, arylene groups and alkyl-aryl-alky groups may optionally be substituted one or several times by a halogen atom, by a nitrile group, or by an alkoxy group. More preferably, R" may be selected from the group consisting of a linear or branched alkylene group having from 1 to 7 carbon atoms, even more preferably, R" may be selected from the group consisting of methylene and ethylene. Preferably, the alcohol of formula (V) can be selected from the group consisting of methylene glycol and ethylene glycol.

If desired, a mixture of alcohols and/or polyol can be applied in a desired molar ratio. In this case, a mixture of the respective carbonates is obtained.

Without departing from the scope of the present invention, instead of or additionally to the alcohol of formula (III) and/or to the polyol of formula (V), a respective alkali metal alcoholate can be used, for example, the respective lithium, sodium, potassium or cesium alcoholate.

The alcoholysis reaction can be performed in the presence of an organic or mineral base like LiF, NaF, KF, CsF, ammonia or a primary, secondary or tertiary amine, e.g. triethylamine or pyridine.

The molar ratio between the alcohol of formula (III) and the fluorosubstituted fluoroformate of the formula (II) preferably is equal to or greater than 0.9:1. Preferably, it is equal to or lower than 5:1. Very good results are achieved when the ratio of alcohol and formate is in the range of 0.95:1 to 1.2:1. The molar ratio between the polyol of formula (V) and the fluorosubstituted fluoroformate of the formula (II) may be adapted by the person skilled in the art according to the number n of OH groups in the polyol (V).

The temperature during alcoholysis is preferably equal to or higher than -80°C, more preferably, equal to or higher than -78°C. The upper temperature can be dependent from pressure and boiling point of the starting materials, e.g. from the boiling point of the alcohol. Often, the temperature is equal to or lower than 85°C.

The reaction can be performed in any suitable reactor, e.g. in an autoclave.

The reaction can be performed batch wise or continuously.

Further details could be found in the International Patent Application WO 2011/006822.

According to a preferred embodiment, the fluorosubstituted (fluoro)alkyl carbonates of the formula (IV) may be selected from the group consisting of 1-fluoroethyl methyl carbonate, 1-fluoroethyl ethyl carbonate, 1-fluoroethyl allyl carbonate, 1-fluoroethyl 2,2,2-trifluoroethyl carbonate, 1-fluoroethyl 2-cyanoethyl carbonate, 1-fluoroethyl methoxyethyl carbonate, 1-fluoroethyl phenyl carbonate, bis(1-fluoroethyl) carbonate, 1-fluoroethyl methyl carbonate, 1-fluoro-1-phenyl-methyl ethyl carbonate and 1-fluoroethyl propargyl carbonate. More preferably, the fluorosubstituted (fluoro)alkyl carbonates of the formula (IV) is 1-fluoroethyl propargyl carbonate.

According to another preferred embodiment, the fluorosubstituted (fluoro)alkyl polycarbonates of the formula (VI) may be the 1,1-di(1-fluoroethyl carbonate) methane and 1,2-di(1-fluoroethyl carbonate) ethane.

According to one object of the present invention, fluorosubstituted (fluoro)alkyl carbamates of the formula (VIII)

FCHR-O-C(O)-NR¹R² (VIII)

may be prepared by reacting a fluorosubstituted fluoroformate of the formula (II) obtained as disclosed above with an with an amine of formula (VII),

R¹R²NH (VII).

R¹ and R², independently from each other, represent a substituted or unsubstituted hydrocarbon group having from 1 to 12 carbon atoms. Preferred amines R¹R²NH of formula (VII) are those wherein R¹ and R² are the same or different and correspond to methyl, ethyl, n propyl and i-propyl.

Similarly, the alcoholysis reaction can be carried out with polyamine instead of an amine. Thus, according to one object of the present invention, fluorosubstituted (fluoro)alkyl polycarbamates of the formula (X)

[FCHR-O-C(O)-NR¹-]ₘ-R"' (X)

may be prepared by reacting a fluorosubstituted fluoroformate of the formula (II) obtained as disclosed above with an with a polyamine of formula (IX),

R"'-[-NR¹H]ₘ (IX).

wherein n is 2, 3 or 4, each R¹, independently from each other, represent a substituted or unsubstituted hydrocarbon group having from 1 to 12 carbon atoms, and R" represents a substituted or unsubstituted hydrocarbon residue having from 1 to 12 carbon atoms. The valence of the hydrocarbon residue R"' depends from the number m: if m is 2, then R"' is a bivalent residue; if m is 3, the R"' is a trivalent residue; if m is 4, then R"' is a tetravalent residue.

The aminolysis reaction may be conducted by the person skilled in the art similarly to the alcoholysis reaction, according to its general knowledge, in view in particular of the International Patent Application WO 2011/006822.

The molar ratio between the amine of formula (VII) and the fluorosubstituted fluoroformate of the formula (II) may preferably be equal to or greater than 0.9:1, and equal to or lower than 5:1, preferably between 0.95:1 to 2.4:1. The molar ratio between the polyamine of formula (IX) and the fluorosubstituted fluoroformate of the formula (II) may be adapted by the person skilled in the art according to the number m of amine groups in the polyamine (IX). The reaction temperature is preferably in the range of 0 to 50°C.

Should the disclosure of any patents, patent applications, and publications which are incorporated herein by reference conflict with the description of the present application to the extent that it may render a term unclear, the present description shall take precedence.

The invention will now be further described in examples without intending to limit it.

### EXAMPLES

### Comparative Example: Preparation of 1-fluoroethyl fluoroformate

Potassium Fluoride (12g; 0,207moles) was suspended in acetonitrile (40g) in a 100ml stainless steel pressure vessel equipped with a Rushton stirrer and temperature measurement under inert conditions (N₂ atmosphere). 1-chloroethyl chloroformate (6g; 0,041 moles); was added via a syringe at room temperature under stirring. The reactor was closed and stirring speed was set and it was heated to a T=100°C for 12h. The reactor was allowed to cool down to ambient temperature. The gas-phase of the reactor was purged to a scrubber system.

A sample of the gas phase was taken for GC. Conversion of 1-chloroethyl chloroformate is 79% and yield of fluoroethyl-fluoroformate is 15%.

### Examples 1 to 3: Preparation of 1-fluoroethyl fluoroformate

The operating procedure is similar to the comparative example, except that potassium bifluoride KHF₂ was added. The test conditions are summarized in the following table. The amount of solvent is fixed to reach a concentration of solids (KF + KHF₂) of 20% w/w of the reaction mixture.

| Example | KF (equivalent) | KHF₂ (equivalent) | Temperature | Fluoroethyl fluoroformate (yield) |
|---|---|---|---|---|
| 1 | 2,17 | 0,97 | 96°C | 89% |
| 2 | 2,32 | 0,26 | 96°C | 42% |
| 3 | 2,29 | 1,01 | 70°C | 91% |

### Example 4: Preparation of 1- fluoroethyl propargyl carbonate

Additional KF was added (1.1 equivalent) to the 1-fluoroethyl fluoroformate obtained in example 3 without further separation, and stirred for 5min. 1-propargyl alcohol (1 equivalent) was added within 5min. It was heated for 9h at 50°C and cooled to room temperature. A quantitative analysis by ¹H NMR shows a global yield of 82% of 1-fluoroethyl prop-2-ynyl carbonate.

### Example 5: Preparation of 1- fluoroethyl phenyl carbonate

Additional KF was added (1.1 equivalent) to the 1-fluoroethyl fluoroformate obtained in example 3 without further separation, and stirred for 5min. Phenol (1 equivalent) was added within 5min. It was heated for 6h at 50°C and cooled to room temperature. A quantitative analysis by ¹H NMR shows a global yield of 79% of 1-fluoroethyl phenyl carbonate.

## Claims

1. A process for preparing fluorosubstituted fluoroformate of the formula (II)
FCHR-O-C(O)F (II)
from halosubstituted haloformate of formula (I)
XCHR-O-C(O)X (I)
wherein R represents a hydrogen atom or a substituted or unsubstituted hydrocarbon group having from 1 to 6 carbon atoms, and each X independently represents a halogen atom, with the proviso that at least one X is different from fluorine,
by reacting said halosubstituted haloformate of formula (I) with at least one fluorinating agent, said fluorinating agent being selected from the group consisting of metal fluorides or organic fluorides, in the presence of a catalyst, said catalyst being selected from the group consisting of metal bifluorides or organic bifluorides.

2. The process according to Claim 1, wherein the halosubstituted haloformate of formula (I) is selected from the group consisting of 1-chloroethyl chloroformate (CH₃CHCl-O-C(O)Cl), 1-chloromethyl chloroformate (CH₂Cl-O-C(O)Cl), and 1-chlorophenyl chloroformate (C₆H₅-CHCl-O-C(O)Cl), and more preferably, the halosubstituted haloformate of formula (I) is 1-chloroethyl chloroformate.

3. The process according to Claim 1 or Claim 2, wherein the fluorinating agent is selected from the group consisting of LiF, KF, CsF, NaF, MgF₂ and CaF₂, preferably from the group consisting of KF and NaF, and more preferably, the fluorinating agent is KF.

4. The process according to any one of Claims 1 to 3, wherein the catalyst is selected from the group consisting of KHF₂, and NaHF₂, and preferably the catalyst is KHF₂.

5. The process according to any one of Claims 1 to 4, wherein the catalyst is prepared in situ by reaction of the fluorinating agent with a Brönsted acid.

6. The process according to any one of Claims 1 to 5, wherein the reaction is carried out with from 1 to 10 molar equivalents, preferably with from 1.1 to 5 molar equivalents, of fluorinating agent.

7. The process according to any one of Claims 1 to 6, wherein the reaction is carried out with from 0.1 to 1.1 molar equivalent, preferably with from 0.25 to 1 molar equivalent, of catalyst.

8. The process according to any one of Claims 1 to 7, wherein the reaction temperature is be equal or higher than the ambient temperature, more preferably equal or higher than 50°C, even more preferably equal or higher than 70°C; and equal to or lower than 300°C, more preferably equal or lower than 200°C, more preferably equal or lower than 100°C.

9. The process according to any one of Claims 1 to 8, wherein the reaction pressure may preferably be equal or higher than 1 bar (abs.), more preferably equal or higher than 1.5 bar (abs.); and equal to or lower than 10 bar (abs.).

10. The process according to any one of Claims 1 to 9, wherein the halosubstituted halo formate of formula (I) is 1-chloroethyl chloroformate, the fluorosubstituted fluoroformate of the formula (II) is 1-fluoroethyl fluoroformate, and the process for preparing 1-fluoroethyl fluoroformate from 1-chloroethyl chloroformate consists in reacting said 1-chloroethyl chloroformate with KF, in the presence of KHF₂.

11. A process for preparing fluorosubstituted (fluoro)alkyl carbonates of the formula (IV)
FCHR-O-C(O)-OR' (IV)
comprising preparing fluorosubstituted fluoroformate of the formula (II) as described in anyone of claims 1 to 10, and reacting said fluorosubstituted fluoroformate of the formula (II) with an alcohol of formula (III)
R'OH (III)
wherein R' represents a substituted or unsubstituted hydrocarbon group having from 1 to 12 carbon atoms.

12. The process according to Claim 11, wherein the fluorosubstituted (fluoro)alkyl carbonates of the formula (IV) is selected from the group consisting of 1-fluoroethyl methyl carbonate, 1-fluoroethyl ethyl carbonate, 1-fluoroethyl allyl carbonate, 1-fluoroethyl 2,2,2-trifluoroethyl carbonate, 1-fluoroethyl 2-cyanoethyl carbonate, 1-fluoroethyl methoxyethyl carbonate, 1-fluoroethyl phenyl carbonate, bis(1-fluoroethyl) carbonate, 1-fluoroethyl methyl carbonate, 1-fluoro-1-phenyl-methyl ethyl carbonate and 1-fluoroethyl propargyl carbonate.

13. A process for preparing fluorosubstituted (fluoro)alkyl polycarbonates of the formula (VI)
[FCHR-O-C(O)-O-]ₙ-R" (VI)
comprising preparing fluorosubstituted fluoroformate of the formula (II) as described in anyone of claims 1 to 10, and reacting said fluorosubstituted fluoroformate of the formula (II) with a polyol of formula (V)
R"-[-OH]ₙ (V)
wherein n is 2, 3 or 4, and R" represents a substituted or unsubstituted hydrocarbon residue having from 1 to 12 carbon atoms.

14. A process for preparing fluorosubstituted (fluoro)alkyl carbamates of the formula (VIII)
FCHR-O-C(O)-NR¹R² (VIII)
comprising preparing fluorosubstituted fluoroformate of the formula (II) as described in anyone of claims 1 to 10, and reacting said fluorosubstituted fluoroformate of the formula (II) with an with an amine of formula (VII),
R¹R²NH (VII)
wherein R¹ and R², independently from each other, represent a substituted or unsubstituted hydrocarbon group having from 1 to 12 carbon atoms.

15. A process for preparing fluorosubstituted (fluoro)alkyl polycarbamates of the formula (X)
[FCHR-O-C(O)-NR¹-]ₘ-R"' (X)
comprising preparing fluorosubstituted fluoroformate of the formula (II) as described in anyone of claims 1 to 10, and reacting said fluorosubstituted fluoroformate of the formula (II) with an with a polyamine of formula (IX),
R"'-[-NR¹H]ₘ (IX).
wherein n is 2, 3 or 4, each R¹, independently from each other, represent a substituted or unsubstituted hydrocarbon group having from 1 to 12 carbon atoms, and R" represents a substituted or unsubstituted hydrocarbon residue having from 1 to 12 carbon atoms.
